(19) Europäisches Patentamt
European Patent Office
Office européen des brevets

(11) Veröffentlichungsnummer: **0 304 738 B1**

(12) # EUROPÄISCHE PATENTSCHRIFT

(45) Veröffentlichungstag der Patentschrift: **30.03.94**

(51) Int. Cl.[5]: **C07C 69/92**, C07C 69/75, C07C 43/205, C07D 239/26, C07D 239/34, C09K 19/20, C09K 19/34, C09K 19/12, C09K 19/30

(21) Anmeldenummer: **88113111.4**

(22) Anmeldetag: **12.08.88**

(54) **Optisch aktive Flüssigkristallkomponenten.**

(30) Priorität: **25.08.87 CH 3245/87**
**07.07.88 CH 2593/88**

(43) Veröffentlichungstag der Anmeldung:
**01.03.89 Patentblatt 89/09**

(45) Bekanntmachung des Hinweises auf die Patenterteilung:
**30.03.94 Patentblatt 94/13**

(84) Benannte Vertragsstaaten:
**CH DE FR GB IT LI NL SE**

(56) Entgegenhaltungen:
**EP-A- 0 269 062**

**PATENT ABSTRACTS OF JAPAN, Band 12, Nr. 450 (C-547)[3297], 25. November 1988, Seite 75 C 547; & JP-A-63 175 095**

**PATENT ABSTRACTS OF JAPAN, Band 13, Nr. 233 (C-601)[3581], 29. Mai 1989, Seite 81 C 601; & JP-A-1 42 454**

(73) Patentinhaber: **F. HOFFMANN-LA ROCHE AG**
**Postfach 3255**
**CH-4002 Basel(CH)**

(72) Erfinder: **Kelly, Stephen, Dr.**
**3A Salinenstrasse**
**CH-4314 Möhlin(CH)**
Erfinder: **Leenhouts, Frans, Dr.**
**73 Violaweg**
**CH-4303 Kaiseraugst(CH)**
Erfinder: **Villiger, Alois, Dr.**
**5 Im Ettingerhof**
**CH-4055 Basel(CH)**

(74) Vertreter: **Cottong, Norbert A. et al**
**Grenzacherstrasse 124**
**Postfach 3255**
**CH-4002 Basel (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue optisch aktive Verbindungen und deren Verwendung für elektrooptische Zwecke sowie flüssigkristalline Gemische und Flüssigkristallzellen, die diese Verbindungen enthalten.

Es ist bekannt, dass die optischen Charakteristiken von Flüssigkristallzellen temperaturabhängig sind. Beispielsweise nimmt die Schwellenspannung von Drehzellenanzeigen ("twisted nematic liquid crystal display", meist abgekürzt als TN-LCDs bezeichnet) mit steigender Temperatur ab. Diese Abhängigkeit ist vor allem für Multiplexanwendungen von Nachteil.

Anderseits werden Flüssigkristallmaterialien für TN-LCDs häufig mit einer optisch aktiven Komponente versetzt zur Vermeidung der Umkehr der Verdrillungsrichtung ("reverse twist"). Für die Schwellenspannung $V^{p}_{th}$ von TN-LCDs gilt bei Verwendung eines solchen, mit optisch aktiver Komponente dotierten Materials angenähert:

$$V^{p}_{th} \quad V^{o}_{th}\,(1+F\bullet d/|p|)^{1/2} \qquad (1)$$

wobei $V^{o}_{th}$ die Schwellenspannung bei Verwendung des undotierten Materials bezeichnet, d den Plattenabstand (d.h. die Schichtdicke des Flüssigkristallmaterials) bedeutet, |p| den absoluten Wert der natürlichen Ganghöhe (pitch) des dotierten Flüssigkristallmaterials bezeichnet und F eine Funktion der elastischen Konstanten und der Verdrillung in der Zelle ist [Appl. Phys. Lett. 25, 12 (1974) und IEEE Trans. Electron Devices ED-10, 141 (1974)].

Aus Gleichung (1) folgt, dass die Abnahme der Schwellenspannung ganz oder teilweise kompensiert werden kann durch Verwendung von optisch aktiven Dotierstoffen, welche einen mit steigender Temperatur abnehmenden absoluten Wert der Ganghöhe p induzieren. Aehnliche Ueberlegungen gelten auch für Flüssigkristallzellen, wie STN-LCDs ("supertwisted nematic"), SBE-LCDs ("supertwisted birefringence effect"), OMI-LCDs ("optical mode interference") und dergleichen.

Aus DE-A-2827471, Z. Naturforschung 34a, 594 (1979) und Phys. Lett. 78A, 285 (1980) ist bekannt, dass ein mit steigender Temperatur abnehmender Wert von |p| erreicht werden kann durch Zusatz von mindestens zwei geeigneten, optisch aktiven Dotierstoffen in bestimmten Mengenverhältnissen. sofern der eine Dotierstoff in der nematischen Trägersubstanz eine rechtshändige Verdrillung und der andere eine linkshändige Verdrillung erzeugt. Eine solche Dotierung hat jedoch den Nachteil, dass zwei oder mehrere optisch aktive Dotierstoffe benötigt werden und im allgemeinen bereits geringe Abweichungen im Mengenverhältnis zu starken Aenderungen im Temperaturverlauf der Schwellenspannung und der Ganghöhe führen. Zudem sind meist relativ hohe Gesamtkonzentrationen an Dotierstoffen erforderlich.

Einzelverbindungen mit der gewünschten Eigenschaft sind jedoch nur wenige bekannt und die Abnahme von |p| mit steigender Temperatur ist in der Regel auf enge oder ungünstig liegende Temperaturbereiche beschränkt, wodurch die Displayqualität bzw. der ausnützbare Temperaturbereich deutlich vermindert wird. EP-A-0211646 offenbart beispielsweise Verbindungen mit zwei gleichen Chiralitätszentren, welche oberhalb 0 °C die gewünschte Temperaturabhängigkeit aufweisen, aber zwischen etwa -20 °C und 0 °C zu Helixinversion führen.

Die Erfindung betrifft optisch aktive Verbindungen der allgemeinen Formel

$$R^1-\overset{CH_3}{\overset{|}{C^*}}H-O-A^1-Z^1-A^2-\!\!\left(Z^2-A^3\right)_m- X -\!\!\left(CH_2\right)_{2n+1}\!\!-\overset{CH_3}{\overset{|}{C^*}}H-R^2 \qquad I$$

worin $R^1$ und $R^2$ unabhängig voneinander Alkyl mit mindestens 2 Kohlenstoffatomen bezeichnen; $A^1$, $A^2$ und $A^3$ unabhängig voneinander unsubstituiertes oder mit Cyano, Halogen und/oder Methyl substituiertes 1,4-Phenylen, in welchem gegebenenfalls eine oder zwei CH-Gruppen durch Stickstoff ersetzt sind, oder unsubstituiertes oder mit Cyano und/oder Methyl substituiertes trans-1,4-Cyclohexylen, in welchem gegebenenfalls eine oder zwei $CH_2$-Gruppen durch Sauerstoff ersetzt sind, darstellen; $Z^1$ und $Z^2$ unabhängig voneinander eine einfache Kovalenzbindung, $-CH_2CH_2-$, $-OCH_2-$, $-CH_2O-$ oder $-C{\equiv}C-$bedeuten; m für die Zahl 0 oder 1 steht; n eine ganze Zahl von 0 bis 4 bezeichnet; X Sauerstoff oder eine Methylengruppe bedeutet; und C* je ein chirales Kohlenstoffatom darstellt, wobei eines der chiralen Kohlenstoffatome die R-Konfiguration und das andere die S-Konfiguration aufweist.

Die Verbindungen der Formel I besitzen im gesamten, für kommerzielle Anwendungen üblicherweise erforderlichen Temperaturbereich, insbesondere im Bereich von etwa -30 °C bis +80 °C, die gewünschte Temperaturabhängigkeit der Ganghöhe. Sie besitzen oft selbst flüssigkristalline Eigenschaften und sind chemisch stabil und gut mischbar mit bekannten Flüssigkristallmaterialien. Die Verbindungen der Formel I eignen sich daher generell zur Modifizierung der Temperaturabhängigkeit der Ganghöhe, insbesondere aber zur Kompensation der Temperaturabhängigkeit der Schwellenspannung.

Die obige Formel I umfasst sowohl Verbindungen, worin $R^1$-C*H($CH_3$)-O- die R-Konfiguration und -X-$(CH_2)_{2n+1}$-C*H($CH_3$)-$R^2$ die S-Konfiguration aufweist, als auch deren optische Antipoden, worin $R^1$-C*H($CH_3$)-O- die S-Konfiguration und -X-$(CH_2)_{2n+1}$-C*H($CH_3$)-$R^2$ die R-Konfiguration aufweist. Die beiden optischen Isomere besitzen jeweils gleiche Eigenschaften, jedoch mit entgegengesetztem Helixdrehsinn, entgegengesetztem Vorzeichen der optischen Rotation etc.

Der Ausdruck "Alkyl mit mindestens 2 Kohlenstoffatomen" umfasst geradkettige und verzweigte Alkylgruppen, wie Aethyl, Propyl, Isopropyl. Butyl, Isobutyl, 2-Methylbutyl, Pentyl, Hexyl, Heptyl, Octyl, Nonyl, Decyl und dergleichen. Gruppen mit 2 bis 10 Kohlenstoffatomen sind im allgemeinen bevorzugt.

Der Ausdruck "Halogen" umfasst Fluor, Chlor, Brom und Jod, insbesondere Fluor und Chlor.

Der Ausdruck "unsubstituiertes oder mit Cyano, Halogen und/oder Methyl substituiertes 1.4-Phenylen, in welchem gegebenenfalls eine oder zwei CH-Gruppen durch Stickstoff ersetzt sind, " umfasst 1,4-Phenylen, Pyridin-2.5-diyl, Pyrazin-2,5-diyl, Pyrimidin-2,5-diyl und Pyridazin-3,6-diyl sowie davon abgeleitete substituierte Ringe wie 2-Methyl-1.4-phenylen, 2-Fluor-1,4-phenylen, 2-Cyano-1,4-phenylen, 2,3-Dicyano-1.4-phenylen und dergleichen.

Der Ausdruck "unsubstituiertes oder mit Cyano und/oder Methyl substituiertes trans-1.4-Cyclohexylen, in welchem gegebenenfalls eine oder zwei $CH_2$-Gruppen durch Sauerstoff ersetzt sind". umfasst Ringe wie trans-1.4-Cyclohexylen, trans-1.3-Dioxan-2.5-diyl und dergleichen sowie davon abgeleitete substituierte Ringe wie 2-Methyl-trans-1,4-cyclohexylen, 1-Cyano-trans-1,4-cyclohexylen und dergleichen.

Die Abnahme der Ganghöhe |p| mit steigender Temperatur basiert hauptsächlich auf den vorliegenden endständigen Gruppen $R^1$-C*H($CH_3$)-O- und -X-$(CH_2)_{2n+1}$-C*H($CH_3$)-$R^2$ und insbesondere der relativen Konfiguration und der Position der chiralen Kohlenstoffatome. Aenderungen in der zentralen Gruppe haben in der Regel nur einen geringen Einfluss auf die Temperaturabhängigkeit der Ganghöhe. Die zentrale Gruppe $-A^1-Z^1-A^2-(Z^2-A^3-)_m$ in Formel I kann daher die oben genannten, in Flüssigkristallen üblichen Ringe und Brückengruppen aufweisen, ohne den gewünschten Effekt wesentlich zu beeinflussen. Anderseits können je nach Wahl der Ringe und Brückengruppen andere Eigenschaften, wie Mesophase, dielektrische Anisotropie, Viskosität, optische Anisotropie und dergleichen, in einem grossen Bereich variiert werden. Insbesondere kann die zentrale Gruppe gewünschtenfalls jeweils so gewählt werden, dass abgesehen von der Temperaturabhängigkeit der Ganghöhe und der Schwellenspannung die übrigen Eigenschaften eines Gemisches nicht oder nur unwesentlich verändert werden.

Vorzugsweise bedeuten die Gruppen $A^1$, $A^2$ und $A^3$ unabhängig voneinander unsubstituiertes oder mit Cyano, Halogen und/oder Methyl substituiertes 1,4-Phenylen, unsubstituiertes oder mit Cyano und/oder Methyl substituiertes trans-1,4-Cyclohexylen oder eine der Gruppen $A^1$, $A^2$ und $A^3$ auch Pyridin-2,5-diyl, Pyrazin-2,5-diyl, Pyrimidin-2,5-diyl, Pyridazin-3,6-diyl oder trans-1,3-Dioxan-2,5-diyl.

Eine bevorzugte Gruppe von Verbindungen der Formel I sind die optisch aktiven Verbindungen der allgemeinen Formel

$$R^1-\overset{CH_3}{\overset{|}{C^*}}H-O-Phe-Z^1-Phe-(Z^2-A^3)_m-X-(CH_2)_{2n+1}-\overset{CH_3}{\overset{|}{C^*}}H-R^2 \quad II$$

$$R^1-\overset{CH_3}{\overset{|}{C^*}}H-O-Phe-Z^1-Py-X-(CH_2)_{2n+1}-\overset{CH_3}{\overset{|}{C^*}}H-R^2 \quad IIa$$

$$R^1-\overset{CH_3}{\overset{|}{C^*}}H-O-Phe-Z^1-Cy-X-(CH_2)_{2n+1}-\overset{CH_3}{\overset{|}{C^*}}H-R^2 \quad IIb$$

worin Phe unsubstituiertes oder mit Cyano, Halogen und/oder Methyl substituiertes 1,4-Phenylen bezeichnet, Py Pyridin-2,5-diyl, Pyrazin-2,5-diyl, Pyrimidin-2,5-diyl oder Pyridazin-3,6-diyl bezeichnet, Cy unsubstituiertes oder mit Cyano und/oder Methyl substituiertes trans-1,4-Cyclohexylen bezeichnet, und $R^1$, $R^2$, $Z^1$, $Z^2$, $A^3$, X, m, n und C* die obigen Bedeutungen haben.

In den obigen Formeln I und II steht $A^3$ vorzugsweise für 1,4-Phenylen, trans-1,4-Cyclohexylen, Pyridin-2,5-diyl, Pyrazin-2,5-diyl, Pyrimidin-2,5-diyl, Pyridazin-3,6-diyl oder trans-1,3-Dioxan-2,5-diyl, insbesondere für 1,4-Phenylen, trans-1,4-Cyclohexylen oder Pyrimidin-2,5-diyl.

$Z^1$ in obigen Formeln I und II bedeutet vorzugsweise eine einfache Kovalenzbindung. $Z^2$ in obigen Formeln I und II bedeutet vorzugsweise eine einfache Kovalenzbindung $-CH_2CH_2-$, $-CH_2O-$ oder $-OCH_2-$, insbesondere eine einfache Kovalenzbindung.

Py in Formel IIa steht vorzugsweise für Pyrimidin-2,5-diyl und Cy in Formel IIb steht vorzugsweise für trans-1,4-Cyclohexylen.

Besonders bevorzugte Verbindungen sind somit die optisch aktiven Verbindungen der allgemeinen Formel

$$R^1-\overset{CH_3}{\overset{|}{C^*}}H-O-Phe-Z^3-(Phe-Z^4)_m-A^4-X-(CH_2)_{2n+1}-\overset{CH_3}{\overset{|}{C^*}}H-R^2 \quad III$$

worin $Z^3$ eine einfache Kovalenzbindung. bedeutet, $Z^4$ eine einfache Kovalenzbindung, $-CH_2CH_2-$, $-CH_2O-$ oder $-OCH_2-$(insbesondere eine einfache Kovalenzbindung) bezeichnet, $A^4$ 1,4-Phenylen, trans-1,4-Cyclohexylen oder Pyrimidin-2,5-diyl darstellt, und Phe, $R^1$, $R^2$, X, m, n und C* die obigen Bedeutungen haben.

Vorzugsweise steht $Z^1$ und/oder $Z^2$ in den Formeln I und II bzw. $Z^3$ und/oder $Z^4$ in Formel III für eine einfache Kovalenzbindung.

Im allgemeinen sind Verbindungen der Formeln I, II, IIa, IIb und III mit unsubstituierten Ringen bevorzugt (d.h. Phe steht vorzugsweise für 1,4-Phenylen und Cy steht vorzugsweise für trans-1,4-Cyclohexylen). Gewünschtenfalls können jedoch durch Verwendung von Ringen mit Cyano-, Halogen-und/oder Methylsubstituenten die dielektrische Anisotropie, die Mesophase, die Löslichkeit und dergleichen modifiziert werden.

Beispiele von bevorzugten Verbindungen der Formel III sind die optisch aktiven Verbindungen der allgemeinen Formeln

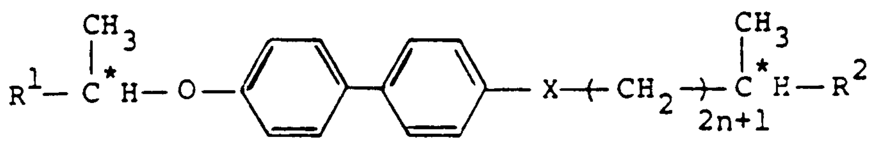 IIIa

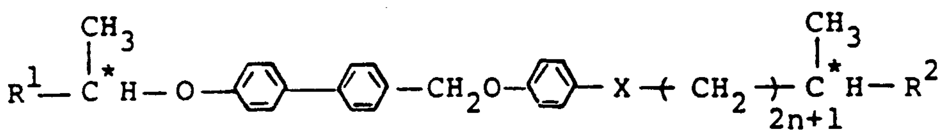 IIIb

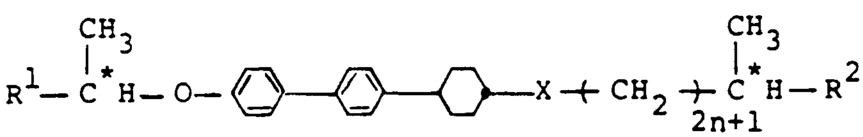 IIIc

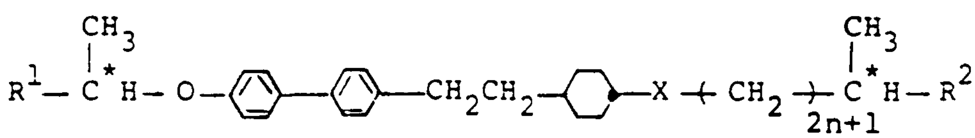 IIId

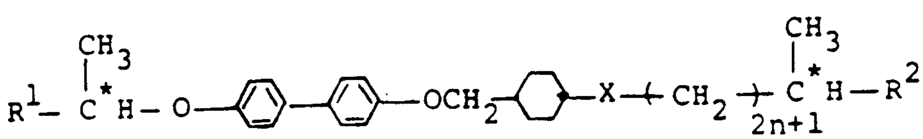 IIIe

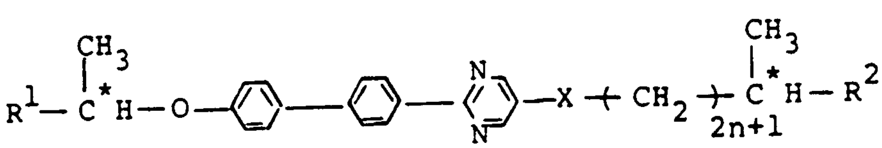 IIIf

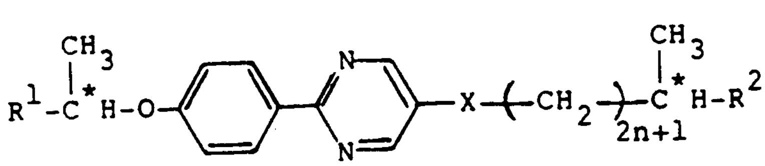 IIIg

worin $R^1$, $R^2$, X, n und C* die obigen Bedeutungen haben.

In obigen Formeln I, II, IIa, IIb, III und IIIa-IIIg steht vorzugsweise $R^1$ für $C_3$-$C_{10}$-Alkyl, insbesondere für geradkettiges Alkyl, wie Propyl, Butyl, Pentyl, Hexyl oder Heptyl, und $R^2$ steht vorzugsweise für $C_2$-$C_6$-

Alkyl, insbesondere für geradkettiges Alkyl, wie Aethyl, Propyl oder Butyl. Besonders bevorzugt sind im allgemeinen diejenigen Verbindungen, worin $R^1$ Hexyl und $R^2$ Aethyl bedeuten. Ferner steht n in den Formeln I-III und IIIa-IIIg vorzugsweise für die Zahl 0. 1 oder 2.

Die erfindungsgemässen Verbindungen können nach an sich bekannten Methoden hergestellt werden. Geeignete Methoden sind dem Fachmann insbesondere von den entsprechenden achiralen Verbindungen sowie von entsprechenden Verbindungen mit einem chiralen Kohlenstoffatom gut bekannt. Aus der Herstellung der letzteren sind auch geeignete Reagenzien zur Einführung der chiralen Endgruppen bekannt.

Die erfindungsgemässen Verbindungen eignen sich insbesondere als Komponenten flüssigkristalliner Gemische. Die Erfindung betrifft daher ebenfalls ein flüssigkristallines Gemisch mit mindestens 2 Komponenten, welches dadurch gekennzeichnet ist, dass mindestens eine Komponente eine optisch aktive Verbindung der Formel I, insbesondere eine der als bevorzugt genannten Verbindungen ist.

Die erfindungsgemässen Verbindungen besitzen in einem weiten Temeraturbereich eine mit steigener Temperatur abnehmende Ganghöhe. Sie können in nematischen, cholesterischen oder smektischen Flüssigkristallmaterialien verwendet werden zur Beeinflussung der Temperaturabhängigkeit der Ganghöhe, insbesondere zur Erzielung einer mit steigender Temperatur abnehmenden Ganghöhe oder beispielsweise in Kombination mit anderen chiralen oder chiral dotierten Materialien zur Erzielung einer im wesentlichen temperaturkonstanten Ganghöhe. Die erfindungsgemässen Verbindungen sind grundsätzlich in allen bekannten Flüssigkristallmaterialien anwendbar.

Der Anteil an Verbindungen der Formel I in den erfindungsgemässen Mischungen kann je nach Anwendungsbereich, Art der weiteren Komponenten, gewünschter Ganghöhe bzw. gewünschter Temperaturabhängigkeit der Ganghöhe etc. in breiten Grenzen variieren und beispielsweise etwa 0.1-50 Gew.-% betragen.

Besonders bevorzugt sind Gemische, welche eine oder mehrere Verbindungen der Formel I und ein nematisches Trägermaterial enthalten. Solche Gemische eignen sich insbesondere zur Temperaturkompensation der Schwellenspannung von Anzeigevorrichtungen mit einer verdrillt nematischen Flüssigkristallschicht. Der Anteil an Verbindungen der Formel I in diesen Gemischen hängt hauptsächlich von der gewünschten Ganghöhe (typischerweise etwa 5-500 $\mu$m) und vom gewünschten Temperaturverlauf der Ganghöhe ab und beträgt im allgemeinen etwa 0.1-10 Gew.-%, beispielsweise etwa 1-5 Gew.-%. Gewünschtenfalls kann das Gemisch einen oder mehrere weitere chirale Dotierstoffe enthalten. Zur Anwendung in TN-LCDs. STN-LCDs, SBE-LCDs oder OMI-LCDs besitzt das Gemisch zweckmässig eine positive dielektrische Anisotropie.

Die Erfindung betrifft ebenfalls eine Anzeigezelle mit diesem Gemisch, nämlich eine Flüssigkristallzelle aus einer zwischen zwei mit Elektroden und Polarisatoren angeordneten, verdrillten nematischen Flüssigkristallschicht, welche dadurch gekennzeichnet ist, dass der Flüssigkristall eine oder mehrere Verbindungen der Formel I enthält. Vorzugsweise besitzt der Flüssigkristall eine positive dielektrische Anisotropie.

Die erfindungsgemässen Gemische und Flüssigkristallzellen können in an sich bekannter Weise hergestellt werden.

Die Erfindung wird durch die folgenden Beispiele weiter veranschaulicht. Die Phasen werden durch folgende Symbole bezeichnet: C für kristallin, $S_C^*$ für chiral smektisch C, Ch für cholesterisch und I für isotrop.

Beispiel 1

In einer Lösung von 5,09 g 4'-[(S)-3-Methylpentyl]-biphenylol (EP-A-131373) in 40 ml Aethanol werden 0,8 g Natriumhydroxid gelöst. Die Lösung wird mit 6,26 g (S)-2-Octyl-p-toluolsulfonat versetzt und 6 Stunden zum Sieden erhitzt. Anschliessend wird das Lösungsmittel abdestilliert und der Rückstand in 100 ml Diäthyläther und 50 ml 0.5N Salzsäure aufgenommen. Die wässrige Phase wird abgetrennt und mit 50 ml Diäthyläther nachextrahiert. Die vereinigte organische Phase wird nacheinander mit 50 ml Wasser, mit 30 ml 3N Natronlauge und dreimal mit je 50 ml Wasser gewaschen, dann über Natriumsulfat getrocknet und eingeengt. Kristallisation des Rückstandes aus Aethanol ergibt 4-[(R)-1-Methylheptyloxy]-4'-[(S)-3-methylpentyl]biphenyl,Smp. (C-I) 12 °C.

In analoger Weise können folgende Verbindungen hergestellt werden:

4-[(R)-1-Methylheptyloxy]-4'-[(S)-2-methylbutyloxy]biphenyl, Smp. (C-I) 42 °C;
4-[(R)-1-Methylheptyloxy]-4'-[(S)-4-methylhexyloxy]biphenyl;
4-[(R)-1-Methylheptyloxy]-4'-[(S)-6-methyloctyloxy]biphenyl;
4-[(R)-1-Methylheptyloxy]-4'-[(S)-8-methyldecyloxy]biphenyl;
4-[(R)-1-Methylheptyloxy]-4'-[(S)-10-methyldodecyloxy]biphenyl;
4-[(R)-1-Methylheptyloxy]-4'-[(S)-5-methylheptyl]biphenyl;

4-[(R)-1-Methylheptyloxy]-4'-[(S)-7-methylnonyl]biphenyl;
4-[(R)-1-Methylheptyloxy]-4'-[(S)-9-methylundecyl]biphenyl;
5-[(S)-2-Methylbutyloxy]-2-(4-[(R)-1-methylheptyloxy]phenyl)pyrimidin;
5-[(S)-4-Methylhexyloxy]-2-(4-[(R)-1-methylheptyloxy]phenyl)pyrimidin;
5-[(S)-6-Methyloctyloxy]-2-(4-[(R)-1-methylheptyloxy]phenyl)pyrimidin;
5-[(S)-8-Methyldecyloxy]-2-(4-[(R)-1-methylheptyloxy]phenyl)pyrimidin;
5-[(S)-10-Methyldodecyloxy]-2-(4-[(R)-1-methylheptyloxy]phenyl)pyrimidin;
5-[(S)-3-Methylpentyl]-2-(4-[(R)-1-methylheptyloxy]phenyl)pyrimidin;
5-[(S)-5-Methylheptyl]-2-(4-[(R)-1-methylheptyloxy]phenyl)pyrimidin, $[\alpha]_D^{22} = +0{,}060°$;
5-[(S)-7-Methylnonyl]-2-(4-[(R)-1-methylheptyloxy]phenyl)pyrimidin;
5-[(S)-9-Methylundecyl]-2-(4-[(R)-1-methylheptyloxy]phenyl)pyrimidin;
sowie die optischen Antipoden der genannten Verbindungen, d.h. diejenigen optischen Isomere, worin jeweils (R) durch (S) und (S) durch (R) ersetzt wird.

Beispiel 2

Ein nematisches Flüssigkristallgemisch (Mischung A) wurde mit verschiedenen Verbindungen der Formel I dotiert und der Temperaturverlauf der natürlichen Ganghöhe p der cholesterischen Gemische untersucht. Die Ergebnisse sind in Tabelle 1 zusammengefasst. Negative Werte der Ganghöhe bezeichnen eine linkshändige Verdrillung und positive Werte eine rechtshändige Verdrillung.

Mischung A:

| | | |
|---|---|---|
| 7,10 | Gew.-% | 4'-Aethyl-4-biphenylcarbonitril, |
| 3,95 | " | 4'-Propyl-4-biphenylcarbonitril, |
| 7,10 | " | 4'-Butyl-4-biphenylcarbonitril, |
| 7,89 | " | 4-(trans-4-Propylcyclohexyl)benzonitril, |
| 15,78 | " | 4-(trans-4-Pentylcyclohexyl)benzonitril, |
| 16,57 | " | 4-Aethoxy-1-[2-(trans-4-propylcyclohexyl)-äthyl]benzol, |
| 4,73 | " | 4-Cyano-4"-pentyl-p-terphenyl, |
| 4,73 | " | 4'-(trans-4-Pentylcyclohexyl)-4-biphenylcarbonitril, |
| 11,05 | " | 4-(trans-4-Pentylcyclohexyl)-1-[2-(trans-4-butylcyclohexyl)äthyl]benzol, |
| 6,42 | " | 4-Aethyl-1-(trans-4-propylcyclohexyl)benzol, |
| 4,59 | " | 4'-(trans-4-Pentylcyclohexyl)-4-[2-(trans-4-butylcyclohexyl)äthyl]biphenyl, |
| 7,34 | " | 4'-(trans-4-Pentylcyclohexyl)-4-[2-(trans-4-butylcyclohexyl)äthyl]-1,1'-äthylendibenzol, |
| 2,75 | " | trans-4-[2-(trans-4-Propylcyclohexyl)äthyl]-cyclohexancarbonsäure-4-cyanophenylester. |

Mischung B:

| | | |
|---|---|---|
| 99 | Gew.-% | Mischung A, |
| 1 | " | 4-[(R)-1-Methylheptyloxy]-4'-[(S)-3-methylpentyl]biphenyl; |

Smp. <-40 °C.

Mischung C:

| | | |
|---|---|---|
| 99 | Gew.-% | Mischung A, |
| 1 | " | 4-[(R)-1-Methylheptyloxy]-4'-[(S)-2-methylbutyloxy]biphenyl; |

Smp. <-40 °C.

Mischung D:

99 Gew.-% Mischung A,
" 5-[(S)-5-Methylheptyl]-2-(4-[(R)-2-methylheptyloxy]phenyl)pyrimidin;

Smp. <-40 °C.

Tabelle 1

| Temperatur (°C) | Ganghöhe p (μm) | | |
|---|---|---|---|
| | Mischung B | Mischung C | Mischung D |
| -40 | - 60,6 | - 63,5 | -111,8 |
| -30 | - 50,2 | - 59,5 | - 82,1 |
| -20 | - 43,3 | - 55,7 | - 65,9 |
| -10 | - 38,5 | - 52,0 | - 55,7 |
| 0 | - 35,0 | - 48,5 | - 48,9 |
| 10 | - 32,3 | - 45,2 | - 43,9 |
| 20 | - 30,3 | - 42,1 | - 40,3 |
| 30 | - 28,7 | - 39,2 | - 37,5 |
| 40 | - 27,5 | - 36,6 | - 35,4 |
| 50 | - 26,6 | - 34,1 | - 33,8 |
| 60 | - 25,9 | - 31,9 | - 32,6 |

## Patentansprüche

1. Optisch aktive Verbindungen der allgemeinen Formel

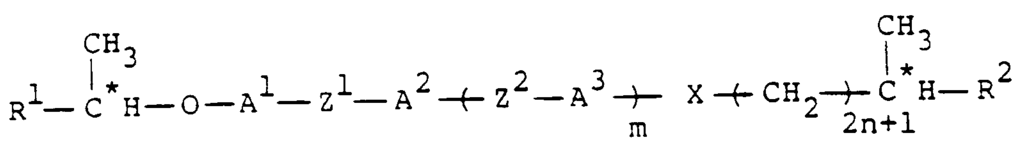

I

worin $R^1$ und $R^2$ unabhängig voneinander Alkyl mit mindestens 2 Kohlenstoffatomen bezeichnen: $A^1$, $A^2$ und $A^3$ unabhängig voneinander unsubstituiertes oder mit Cyano, Halogen und/oder Methyl substituiertes 1,4-Phenylen, in welchem gegebenenfalls eine oder zwei CH-Gruppen durch Stickstoff ersetzt sind, oder unsubstituiertes oder mit Cyano und/oder Methyl substituiertes trans-1,4-Cyclohexylen, in welchem gegebenenfalls eine oder zwei $CH_2$-Gruppen durch Sauerstoff ersetzt sind, darstellen; $Z^1$ und $Z^2$ unabhängig voneinander eine einfache Kovalenzbindung, $-CH_2CH_2-$, $-OCH_2-$, $-CH_2O-$ oder $-C{\equiv}C-$. bedeuten; m für die Zahl 0 oder 1 steht; n eine ganze Zahl von 0 bis 4 bezeichnet; X Sauerstoff oder eine Methylengruppe bedeutet; und C* ein chirales Kohlenstoffatom darstellt, wobei eines der chiralen Kohlenstoffatome die R-Konfiguration und das andere die S-Konfiguration aufweist.

2. Optisch aktive Verbindungen nach Anspruch 1, gekennzeichnet durch die allgemeinen Formeln

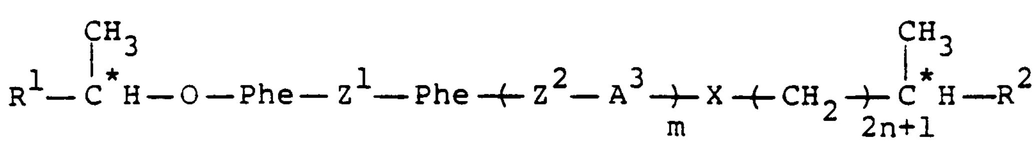

II

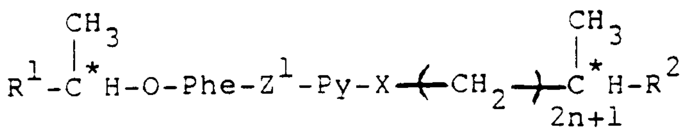

IIa

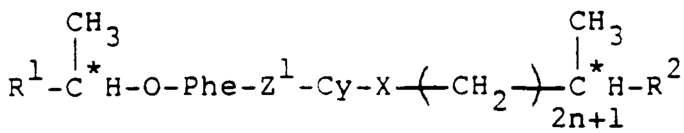

IIb

worin Phe unsubstituiertes oder mit Cyano, Halogen und/oder Methyl substituiertes 1,4-Phenylen bezeichnet, Py Pyridin-2,5-diyl, Pyrazin-2,5-diyl, Pyrimidin-2,5-diyl oder Pyridazin-3,6-diyl bezeichnet, Cy unsubstituiertes oder mit Cyano und/oder Methyl substituiertes trans-1,4-Cyclohexylen bezeichnet, und $R^1$, $R^2$, $Z^1$, $Z^2$, $A^3$, X, m, n und C* die in Anspruch 1 gegebenen Bedeutungen haben.

**3.** Optisch aktive Verbindungen nach Anspruch 1 oder 2, gekennzeichnet durch die allgemeine Formel

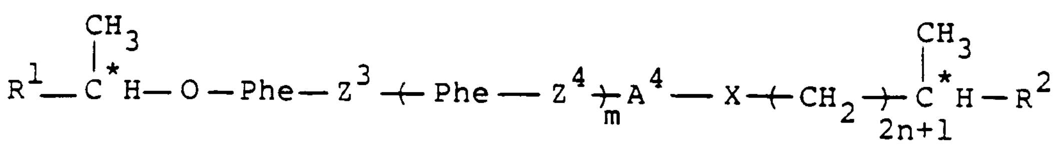

III

worin Phe unsubstituiertes oder mit Cyano, Halogen und/oder Methyl substituiertes 1,4-Phenylen bezeichnet, $Z^3$ eine einfache Kovalenzbindung, bedeutet, $Z^4$ eine einfache Kovalenzbindung, $-CH_2CH_2-$, $-CH_2O-$, oder $-OCH_2-$ bezeichnet, $A^4$ 1,4-Phenylen, trans-1,4-Cyclohexylen oder Pyrimidin-2,5-diyl darstellt, und $R^1$, $R^2$, X, m, n und C* die in Anspruch 1 gegebenen Bedeutungen haben.

**4.** Optisch aktive Verbindungen nach einem der Ansprüche 1 bis 3, dadurch gekennzeichnet, dass $Z^1$ und/oder $Z^2$ bzw. $Z^3$ und/oder $Z^4$ eine einfache Kovalenzbindung bedeutet.

**5.** Optisch aktive Verbindungen nach einem der Ansprüche 1 bis 4, dadurch gekennzeichnet, dass $R^1$ $C_3$-$C_{10}$-Alkyl, vorzugsweise Propyl, Butyl, Pentyl, Hexyl oder Heptyl bezeichnet.

**6.** Optisch aktive Verbindungen nach einem der Ansprüche 1 bis 5, dadurch gekennzeichnet, dass $R^2$ $C_2$-$C_6$-Alkyl, vorzugsweise Aethyl, Propyl oder Butyl bezeichnet.

**7.** Optisch aktive Verbindungen nach einem der Ansprüche 1 bis 6, dadurch gekennzeichnet, dass n für die Zahl 0, 1 oder 2 steht.

**8.** Flüssigkristallines Gemisch mit mindestens 2 Komponenten, dadurch gekennzeichnet, dass mindestens eine Komponente eine optisch aktive Verbindung gemäss Ansprüchen 1 bis 7 ist.

**9.** Flüssigkristallines Gemisch nach Anspruch 8, dadurch gekennzeichnet, dass es eine oder mehrere optisch aktive Verbindungen gemäss Ansprüchen 1 bis 7 und ein nematisches Trägermaterial enthält.

**10.** Flüssigkristallzelle aus einer zwischen zwei mit Elektroden und Oberflächenorientierungen versehenen Platten und Polarisatoren angeordneten, verdrillten nematischen Flüssigkristallschicht, dadurch gekennzeichnet, dass der Flüssigkristall eine oder mehrere Verbindungen gemäss Ansprüchen 1 bis 7 enthält.

**11.** Verwendung der Verbindungen gemäss Ansprüchen 1 bis 7 für elektro-optische Zwecke.

**12.** Verwendung der Verbindungen gemäss Ansprüchen 1 bis 7 zur Kompensation der Temperaturabhängigkeit der Schwellenspannung von Flüssigkristallzellen.

## Claims

**1.** Optically active compounds of the general formula

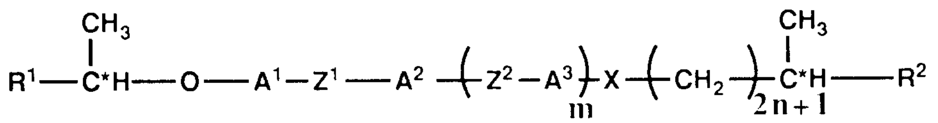
I

wherein $R^1$ and $R^2$ each independently denote alkyl with at least 2 carbon atoms; $A^1$, $A^2$ and $A^3$ each individually represent unsubstituted or cyano-, halogen- and/or methyl-substituted 1,4-phenylene, in which optionally one or two CH groups is/are replaced by nitrogen, or unsubstituted or cyano- and/or methyl-substituted trans-1,4-cyclohexylene, in which optionally one or two $CH_2$ groups is/are replaced by oxygen; $Z^1$ and $Z^2$ each independently signify a single covalent bond, $-CH_2CH_2-$, $-OCH_2-$, $-CH_2O-$ or $-C{\equiv}C-$; m stands for the number 0 or 1; n denotes a whole number of 0 to 4; X signifies oxygen or a methylene group; and each C* represents a chiral carbon atom, whereby one of the chiral carbon atoms has the R-configuration and the other has the S-configuration.

**2.** Optically active compounds according to claim 1, characterized by the general formulae

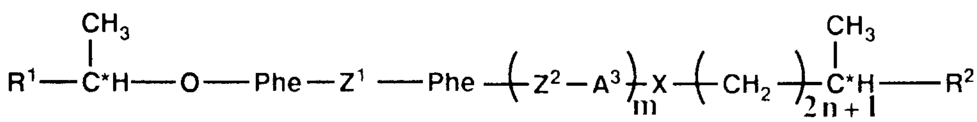
II

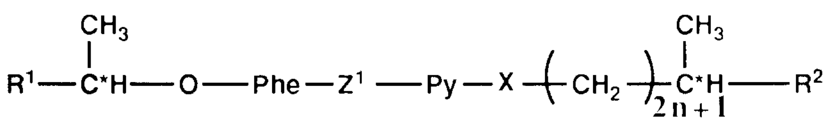
IIa

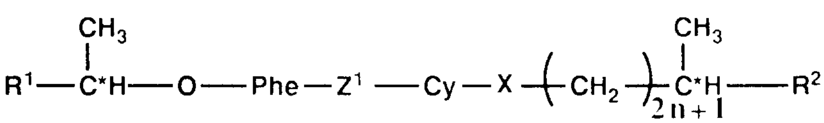
IIb

wherein Phe denotes unsubstituted or cyano-, halogen-and/or methyl-substituted 1,4-phenylene, Py denotes pyridine-2,5-diyl, pyrazine-2,5-diyl, pyrimidine-2,5-diyl or pyridazine-3,6-diyl, Cy denotes unsubstituted or cyano- and/or methyl-substituted trans-1,4-cyclohexylene and $R^1$, $R^2$, $Z^1$, $Z^2$, $A^3$, X, m, n and C* have the significances given in claim 1.

**3.** Optically active compounds according to claim 1 or 2, characterized by the general formula

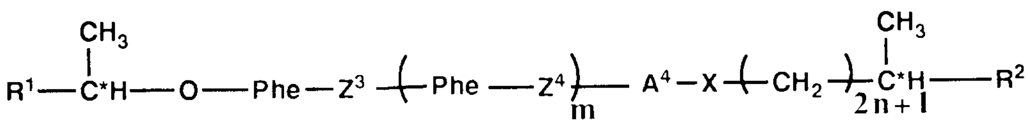
III

wherein Phe denotes unsubstituted or cyano-, halogen-and/or methyl-substituted 1,4-phenylene, $Z^3$ signifies a single covalent bond, $Z^4$ denotes a single covalent bond, $-CH_2CH_2-$, $-CH_2O-$ or $-OCH_2-$, $A^4$ represents 1,4-phenylene, trans-1,4-cyclohexylene or pyrimidine-2,5-diyl and $R^1$, $R^2$, X, m, n and C* have the significances given in claim 1.

4. Optically active compounds according to any one of claims 1 to 3, characterized in that $Z^1$ and/or $Z^2$ or $Z^3$ and/or $Z^4$ signify a single covalent bond.

5. Optically active compounds according to any one of claims 1 to 4, characterized in that $R^1$ denotes $C_3$-$C_{10}$-alkyl, preferably propyl, butyl, pentyl, hexyl or heptyl.

6. Optically active compounds according to any one of claims 1 to 5, characterized in that $R^2$ denotes $C_2$-$C_6$-alkyl, preferably ethyl, propyl or butyl.

7. Optically active compounds according to any one of claims 1 to 6, characterized in that n stands for the number 0, 1 or 2.

8. A liquid crystalline mixture having at least 2 components, characterized in that at least one component is an optically active compound in accordance with claims 1 to 7.

9. A liquid crystalline mixture according to claim 8, characterized in that it contains one or more optically active compounds in accordance with claims 1 to 7 and a nematic carrier material.

10. A liquid crystal cell comprising a twisted nematic liquid crystal layer arranged between two plates, provided with electrodes and surface orientations, and polarizers, characterized in that the liquid crystal contains one or more compounds in accordance with claims 1 to 7.

11. The use of the compounds in accordance with claims 1 to 7 for electro-optical purposes.

12. The use of the compounds in accordance with claims 1 to 7 for compensating the temperature dependence of the threshold potential of liquid crystal cells.

**Revendications**

1. Composés optiquement actifs de formule générale

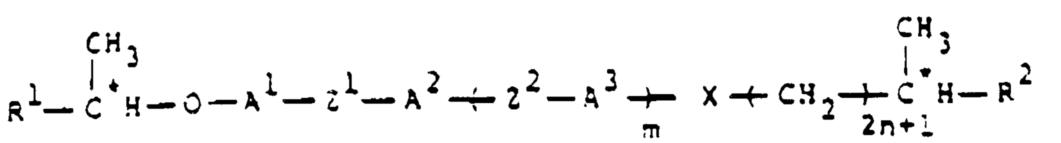 I

dans laquelle $R^1$ et $R^2$ représentent un alkyle indépendamment avec au moins 2 atomes de carbone ; $A^1$, $A^2$ et $A^3$ représentent indépendamment l'un de l'autre un 1,4-phénylène non substitué ou substitué par cyano, halogène et/ou méthyle, dans lequel le cas échéant un ou deux groupes CH sont remplacés par l'azote, ou un trans-1,4-cyclohexylène non substitué ou substitué par cyano et/ou méthyle, dans lequel le cas échéant un ou deux groupes $-CH_2$ sont remplacés par l'oxygène ; $Z^1$ et $Z^2$ indépendamment l'un de l'autre représentent une liaison covalente simple, $-CH_2CH_2-$, $-OCH_2-$, $-CH_2O-$ ou $-C{\equiv}C-$ ; m est le nombre 0 ou 1 ; n est un nombre entier de 0 à 4 ; X représente l'oxygène ou un groupe méthyle ; et C* représente un atome de carbone chiral, l'un des atomes de carbone chiraux présentant la configuration R et l'autre la configuration S.

2. Composés optiquement actifs selon la revendication 1, caractérisés par les formules générales

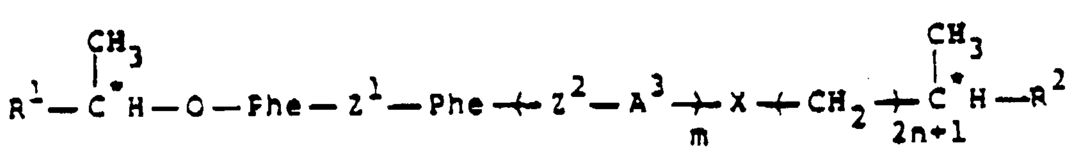 II

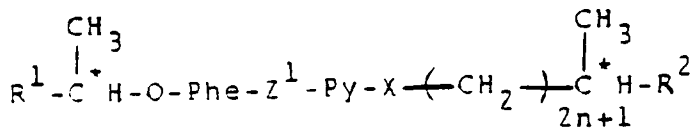 IIa

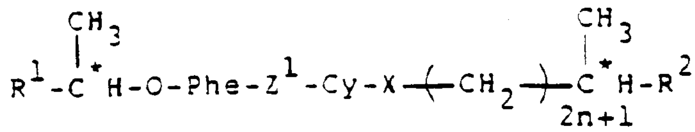 IIb

dans lesquelles Phe représente un 1,4-phénylène non substitué ou substitué par cyano, halogène et/ou méthyle, Py un pyridin-2,5-diyle, pyrazin-2,5-diyle, pyrimidin-2,5-diyle ou pyridazin-3,6-diyle, Cy un trans-1,4-cyclohexylène non substitué ou substitué par cyano et/ou méthyle, et $R^1$,$R^2$,$Z^1$,$Z^2$,$A^3$,X,m,n et $C^*$ ont les significations données à la revendication 1.

**3.** Composés optiquement actifs selon la revendication 1 ou 2, caractérisés par la formule générale

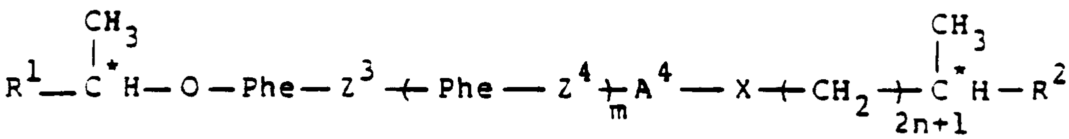 III

dans laquelle Phe représente un 1,4-phénylène substitué par cyano halogène et/ou méthyle, $Z^3$ représente une liaison covalente simple, $Z^4$ représente une liaison covalente simple, $-CH_2CH_2-$, $-CH_2O-$ ou $-OCH_2-$, $A^4$ représente un 1,4-phénylène, trans-1,4-cyclohexylène ou pyrimidin-2,5-diyle, et $R^1$,$R^2$,X,m,n et $C^*$ ont les significations données à la revendication 1.

**4.** Composés optiquement actifs selon l'une des revendications 1 à 3, caractérisés en ce que $Z^1$ et/ou $Z^2$ ou $Z^3$ et/ou $Z^4$ représentent une liaison covalente simple.

**5.** Composés optiquement actifs selon l'une des revendications 1 à 4, caractérisés en ce que $R^1$ représente un alkyle $C_3$-$C_{10}$, de préférence le propyle, butyle, pentyle, hexyle ou heptyle.

**6.** Composés optiquement actifs selon l'une des revendications 1 à 5, caractérisés en ce que $R^2$ représente un alkyle $C_2$-$C_6$, de préférence l'éthyle, propyle ou butyle.

**7.** Composés optiquement actifs selon l'une des revendications 1 à 6, caractérisés en ce que n représente les nombres 0, 1 ou 2.

**8.** Mélange de cristaux liquides avec au moins 2 composants, caractérisé en ce qu'au moins un composant est un composé optiquement actif selon les revendications 1 à 7.

**9.** Mélange de cristaux liquides selon la revendication 8, caractérisé en ce qu'il contient un ou plusieurs composés optiquement actifs selon les revendications 1 à 7 et un matériau support nématique.

**10.** Cellule de cristaux liquides constituée d'une couche de cristaux liquides nématiques câblée disposée entre deux plaques et polariseurs munis d'électrodes et d'orientateurs de surface caractérisée en ce que le cristal liquide contient un ou plusieurs composés selon les revendications 1 à 7.

**11.** Utilisation des composés selon les revendications 1 à 7, pour des utilisations électro-optiques.

**12.** Utilisation des composés selon les revendications 1 à 7 pour compenser la sensibilité à la température des tensions de seuil des cellules de cristaux liquides.